# EUROPEAN PATENT SPECIFICATION

(11) **EP 4 084 741 B1**
(45) Date of publication and mention of the grant of the patent: **03.07.2024**
(21) Application number: 21712687.9
(22) Date of filing: 25.02.2021
(51) Int. Cl.: A61F 2/24

(54) **SUTURE WITH APERTURE FOR USE IN PROSTHETIC DEVICES**
NAHT MIT ÖFFNUNG ZUR VERWENDUNG IN PROTHETISCHEN VORRICHTUNGEN
SUTURE AVEC OUVERTURE DESTINÉE À ÊTRE UTILISÉE DANS DES DISPOSITIFS PROTHÉTIQUES

(30) Priority: 04.03.2020 US 202062984959 P
(43) Date of publication of application: 09.11.2022
(73) Proprietor: Edwards Lifesciences Corporation, Irvine, CA 92614-5688 (US)
(72) Inventor: GOLDBERG, Eran, 30889 Caesarea (IL); NIR, Noam, 30889 Caesarea (IL)
(74) Representative: Venner, Julia Ann
(86) International application number: PCT/US2021/019681
(87) International publication number: WO 2021/178216

(56) References cited:
- WO-A2-2015/109027
- US-A- 2 451 355
- US-A1- 2018 028 310
- US-A1- 2018 133 003
- US-A1- 2018 325 665

## Description

### CROSS-REFERENCE TO RELATED APPLICATIONS

This application claims the benefit of U.S. Provisional Application No. 62/984,959, filed March 4, 2020.

### FIELD

The present disclosure concerns aspects of prosthetic valves and sutures used to provide enhanced structural integrity and strength thereto.

### BACKGROUND

The human heart can suffer from various valvular diseases. These valvular diseases can result in significant malfunctioning of the heart and ultimately require the replacement of the native valve with an artificial valve.

When the native valve is replaced, surgical implantation of a prosthetic valve had required an open-chest surgery during which the heart is stopped, and the patient is placed on cardiopulmonary bypass (a so-called "heart-lung machine"). Because of the drawbacks associated with conventional open-heart surgery, percutaneous and minimally-invasive surgical approaches are garnering intense attention. In one technique, a prosthetic valve is configured to be implanted in a much less invasive procedure by way of catheterization. For instance, U.S. Pat. Nos. 5,411 ,522 and 6,730,118, describe collapsible transcatheter heart valves that can be percutaneously introduced in a compressed state on a catheter and expanded in the desired position by balloon inflation or by utilization of a self-expanding frame or stent.

An important design parameter of a transcatheter heart valve is to ensure the integrity of all valve components. Many of the prosthetic valve components are connected with various sutures. Similar sutures are also used to connect the valve's components with the surrounding tissues as appropriate. One drawback of this structure is that the suture can cut through the tissue causing unnecessary damage to the valve resulting in valve malfunction. Therefore, there are needs for reinforcement members and methods for attaching the same that substantially eliminate these issues and improve the safety of the patient. These needs and other needs are at least partially satisfied by the present disclosure.

US 2018/0325665 describes a prosthetic heart valve including an annular frame including a plurality of strut members that is radially collapsible and expandable. A leaflet structure is situated within the frame, and includes a plurality of leaflets having opposing commissure tab portions on opposite sides of the leaflet. To assemble each commissure, each upper tab is folded along a horizontal fold line to form first and second tab layers, and a first vertically extending reinforcing member can be placed against he first tab layer adjacent its inner edge. A second vertically extending reinforcing member can be placed against he second tab layer opposite the first reinforcing member.

US 2018/0028310 describes a prosthetic heart valve including a radially collapsible and expandable annular frame having a plurality of struts defining openings. The prosthetic heart valve can further include a plurality of leaflets that regulate the flow of blood through the frame. A reinforcing member such as a wire, chord, sleeve, fabric or suture can be placed along the upper surface of the leaflet at the bending axis where the lower edge portion intersects with the articulating portion of the leaflet.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention

The invention is directed to a prosthetic heart valve as defined in independent claim 1.

One aspect of the prosthetic valve can comprise the attachment member that comprises one or more stitches. In certain aspects, the stitches are used to couple the suture to the prosthetic valve. In such aspects, the stitches pass through the plurality of apertures present in the at least one suture and couple the suture to the device.

The invention is also directed to a method of making a prosthetic heart valve as defined in independent claim 15.

Also disclosed herein is a kit comprising: at least one suture having a longitudinal axis and a transverse axis perpendicular to the longitudinal axis, and wherein at least a portion of the suture comprises a plurality of apertures, wherein each of the plurality of apertures is configured to receive an attachment member for coupling the suture to an article. In these aspects, the first attachment member can comprise one or more stitches, flexible connectors, or any combination thereof.

The foregoing and other features and advantages of the disclosure will become more apparent from the following detailed description, which proceeds with reference to the accompanying figures.

### BRIEF DESCRIPTION OF THE DRAWINGS

**FIGURES 1A-1B** show a top flat-view of exemplary sutures; **FIGURE 1C** shows a photograph of an exemplary suture.
**FIGURE 2** shows a front view of an exemplary aspect of a prosthetic heart valve.
**FIGURE 3** shows a side view of the prosthetic heart valve of **FIG. 2** with the outer skirt removed to show an inner skirt and valvular structure mounted on the frame.
**FIGURE 4** shows a rotated front view of the prosthetic heart valve of **FIG. 3****.**
**FIGURES 5A-5B** show an enlarged, partial cross-sectional view of a prosthetic heart valve, according to some aspects.
**FIGURE 6** shows an enlarged, partial cross-sectional view of a prosthetic heart valve, according to one aspect.
**FIGURE 7** shows a top plan view of the prosthetic heart valve of **FIG. 2****.**
**FIGURE 8** shows a side view of an exemplary leaflet shown in a flattened configuration.
**FIGURE 9** shows a perspective view of an exemplary frame of the prosthetic heart valve of **FIG. 2****.**
**FIGURE 10** shows a perspective view of an exemplary frame of the prosthetic heart valve of **FIG. 2****.**

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "suture" includes aspects having two or more such sutures unless the context clearly indicates otherwise.

It is also to be understood that the terminology used herein is for the purpose of describing particular aspects only and is not intended to be limiting. As used in the specification and in the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

Ranges can be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It should be further understood that the endpoints of each of the ranges are significant both in relation to the other endpoint, and independently of the other endpoint.

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

Further, the terms "coupled" and "associated" generally means electrically, electromagnetically, and/or physically (e.g., mechanically or chemically) coupled or linked and does not exclude the presence of intermediate elements between the coupled or associated items.

As used herein, the term "suture" refers to a multifilament thread, where the filaments are braided together to form a thread having predetermined dimensions. In certain aspects, the suture described herein can comprise two or more threads (cords) that are fused together. It is understood that the sutures of the present disclosure do not comprise woven or nonwoven fabric.

As used herein, the term "stitch" refers to a monofilament or multifilament thread that is used as an attachment member. Some exemplary stitching sutures can have dimensions from 6-0 to 4-0.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

In one aspect disclosed herein is a prosthetic valve comprising: at least one reinforcement member configured to be attached with a first attachment member to one or more components of the prosthetic valve; wherein the at least one reinforcement member comprises a suture having a longitudinal axis and a transverse axis perpendicular to the longitudinal axis, and wherein at least a portion of the suture comprises a plurality of apertures, wherein each of the plurality of apertures is configured to receive the first attachment member for coupling the suture to the prosthetic valve. It is understood, however, that the suture, as disclosed herein, does not have to function as a reinforcement member but can be used as a member configured to couple various components of the prosthetic valve or a simple article.

In certain aspects and as described herein, the suture comprises a multifilament thread. In such aspects, the thread comprises two or more filaments wherein the filaments have a thickness from about 0.02 mm to about 0.5 mm, including exemplary values of about 0.03 mm, about 0.04 mm, about 0.05 mm, about 0.06 mm, about 0.07 mm, about 0.1 mm, about 0.12 mm, about 0.15 mm, about 0.17 mm, about 0.2 mm, about 0.22 mm, about 0.25 mm, about 0.27 mm, about 0.3 mm, about 0.32 mm, about 0.35 mm, about 0.37 mm, about 0.4 mm, about 0.42 mm, about 0.45 mm, and about 0.47 mm. It is further understood that each of the filaments in the thread can have the same thickness or different.

In still further aspects, the suture can comprise two or more threads that are coupled together. In such exemplary aspects, each thread can comprise two or more filaments, as disclosed above. In yet further aspects, the suture can have a width from about 0.1 mm to about 10 mm, including exemplary values about 0.2 mm, about 0.3 mm, about 0.4 mm, about 0.5 mm, about 0.6 mm, about 0.7 mm, about 0.8 mm, about 0.9 mm, about 1.0 mm, about 1.2 mm, about 1.5 mm, about 1.8 mm, about 2 mm, about 2.2 mm, about 2.5 mm, about 2.8 mm, about 3 mm, about 3.2 mm, about 3.5 mm, about 3.8 mm, about 4 mm, about 4.2 mm, about 4.5 mm, about 4.8 mm, about 5 mm, about 5.2 mm, about 5.5 mm, about 5.8 mm, about 6 mm, about 6.2 mm, about 6.5 mm, about 6.8 mm, about 7 mm, about 7.2 mm, about 7.5 mm, about 7.8 mm, about 8 mm, about 8.2 mm, about 8.5 mm, about 8.8 mm, about 9 mm, about 9.2 mm, about 9.5 mm, and about 9.8 mm. It is understood that the width of the suture can have any value between any two foregoing values.

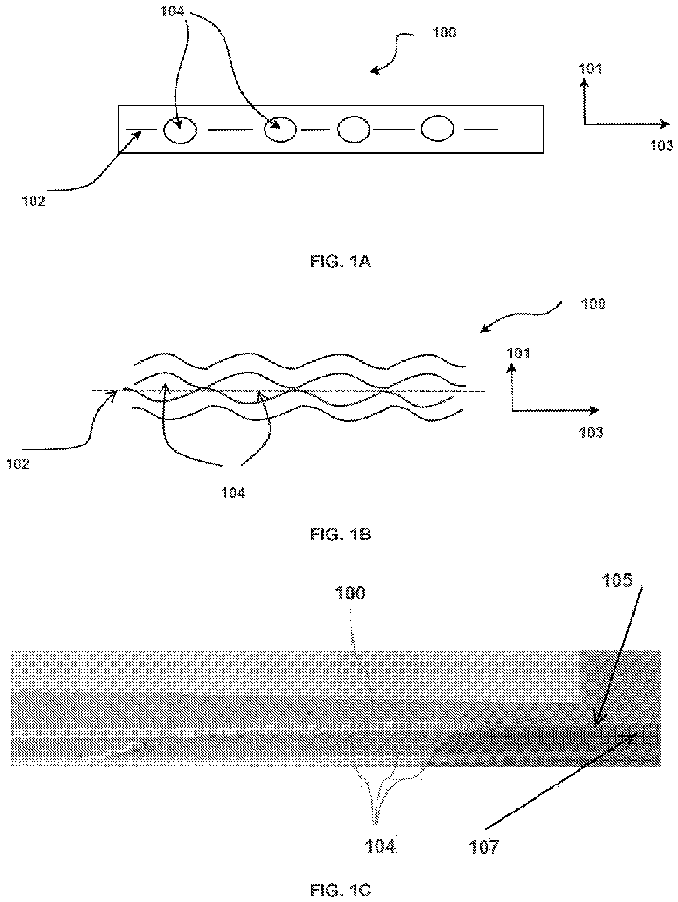

**FIGS 1A-1C** show exemplary aspects of a reinforcement member comprising a suture **100.** **FIG. 1A** and **1B****,** for example, show the suture **100,** having a longitudinal axis **103** and transverse axis **101.** The suture **100** also comprises a centerline **102.** Exemplary suture **100** in **FIG. 1A** is a multifilament thread (cord) having a rectangular shape and having a plurality of apertures **104** disposed along at least a portion of the centerline **102.** The plurality of apertures **104,** which forms an opening extending through all or a portion of the suture **100.** **FIG. 1B** shows a braided suture **100** having a plurality of apertures **104** braided within the suture **100.** **FIG. 1C** shows a photograph of the exemplary suture **100** comprising two separate braided threads **105** and **107** fused together to form a plurality of apertures **104.**

Apertures **104,** in certain aspects, can be positioned within at least a portion of the centerline **102.** That is, the opening formed by the apertures **104** is positioned laterally (i.e., along a transverse axis **101** of the suture **100**) along at least a portion of the centerline **102,** along all or a portion of the entire length of the suture **100.** For example, the apertures **104** can be centered along the centerline **102** of the suture **100.** That is, the lateral center of the aperture **104** is aligned with the centerline **102.** Yet, in other aspects, the plurality of apertures **104** can be located along the centerline **102** such that the center of the aperture **104** is not aligned with the centerline **102.** For example, the lateral center of each (or a subset) of the plurality of apertures **104** is not aligned with the centerline **102** of the suture **100,** e.g., offset from the centerline **102** along the transverse axis **101** of the suture **100.** In further aspects, all or a portion of the plurality of apertures **104** are located along the suture **100** such that the aperture **104** is not aligned with the centerline **102.** In such exemplary aspects, some of the plurality of apertures are located along the suture **100** such that the aperture **104** is not aligned with the centerline **102,** while others are aligned. In some exemplary aspects, no portion of the opening overlaps/aligns with centerline **102** of the suture **100.**

In certain aspects and without any limitations, apertures **104** present in a suture **100** can have any design that would allow the desired outcome. For example, apertures **104** can have any regular or irregular shape, including, for example, a curvilinear (e.g., circular, elliptical, egg-shaped) or rectilinear shape (e.g., square, rectangle, trapezoid, rhombus, triangle, star). Various apertures of a plurality of apertures can have the same or different shapes. In certain aspects, a plurality of apertures **104** can be positioned in one longitudinally-extending row along a length of the suture **100,** while in other aspects, a plurality of apertures **104** can be positioned in two or more longitudinally-extending rows along a length of the suture **100.** In certain aspects, if a plurality of apertures **104** are positioned in two or more longitudinally-extending rows, the apertures **104** can be positioned on the same line along the transverse axis, or they can be shifted/offset relative to each other. It is understood that the present disclosure does not limit the design and specific positioning of the plurality of apertures **104** in the suture **100.**

Various apertures of the plurality of apertures **104** within the suture **100** can have the same size or can have different sizes. The size of each of the plurality of apertures **104** is substantially large enough to allow a needle and a stitch to pass through. In certain aspects and without limitations, the size of each of the plurality of apertures **104** is from about 0.001 mm to about 1 cm, including exemplary values of about 0.002 mm, about 0.005 mm, about 0.008 mm, about 0.01 mm, about 0.02 mm, about 0.05 mm, about 0.08 mm, about 0.1 mm, about 0.2 mm, about 0.5 mm, about 0.8 mm, about 1.0 mm, about 1.2 mm, about 1.5 mm, about 1.8 mm, about 2.0 mm, about 2.2 mm, about 2.5 mm, about 2.8 mm, about 3.0 mm, about 3.2 mm, about 3.5 mm, about 3.8 mm, about 4.0 mm, about 4.2 mm, about 4.5 mm, about 4.8 mm, about 5.0 mm, about 5.2 mm, about 5.5 mm, about 5.8 mm, about 5.0 mm, about 5.2 mm, about 5.5 mm, about 5.8 mm, about 6.0 mm, about 6.2 mm, about 6.5 mm, about 6.8 mm, about 7.0 mm, about 7.2 mm, about 7.5 mm, about 7.8 mm, about 8.0 mm, about 8.2 mm, about 8.5 mm, about 8.8 mm, about 9.0 mm, about 9.2 mm, about 9.5 mm, and about 9.8 mm. For example, and without limitations, in some aspects, a portion of the plurality of apertures **104** in the suture **100** can have a size different from another portion of the plurality of apertures **104.** Even in further aspects, two adjacent apertures **104** in the plurality of apertures **104** can have the same size or different. It is understood that the size, as defined herein, references to diameter or to the largest dimensional length depending on the specific shape and type of the aperture **104.**

In still further aspects, the apertures of a plurality of apertures **104** are spaced apart by a spacing increment *d* along a length of the suture **100.** The spacing *d* measured between the proximal end of the opening of a first aperture and a distal end of the opening of an adjacent/second aperture. It is understood that in some aspects, the spacing increment *d* between any two adjacent apertures of the plurality of apertures **104** is consistent amongst the plurality. While in other aspects, the spacing increment *d* between any two adjacent apertures of the plurality of apertures **104** varies amongst the plurality. In still further aspects, the spacing increment *d* can have any value that is substantial enough to ensure the structural integrity of the suture **100.** In certain aspects, the spacing increment *d* between any two adjacent apertures is at least about 5% of the largest size aperture of the plurality of apertures present in the suture, at least about 10%, at least about 15%, at least about 20%, at least about 30%, at least about 40%, at least about 50%, about least about 60%, at least about 70%, at least about 80%, at least about 90%, at least about 100%, or at least about 150% of the largest size aperture of the plurality of apertures present in the suture.

It is understood that the disclosed herein suture **100** is configured to be attached with the first attachment member and/or second attachment member to one or more components of the prosthetic valve. The first attachment member or the second attachment member can be any member configured to permanently, semi-permanently, and/or removably attach the suture **100** to the prosthetic valve. In certain aspects, the first attachment member and the second attachment member are the same, while in other aspects, they are different. In certain aspects, the first attachment member (and/or second attachment member) comprises stitches. In yet other aspects, the first attachment member (and/or second attachment member) comprises any flexible connectors known in the art and applicable to the desired application. The stitches (or any other attachment members as known in the art) pass through one or more apertures to couple the suture to the prosthetic valve. In certain aspects, a needle can be used to pass the stitches (or any other attachment members as known in the art) through the one or more apertures **104.** In certain aspects, the stitches comprise a mono or multifilament thread comprising filaments having a thickness from 6-0 to 4-0. In yet other aspects, the stitches comprise a mono or multifilament thread comprising filaments having a thickness of from about 0.02 mm to about 0.5 mm, including exemplary values of about 0.03 mm, about 0.04 mm, about 0.05 mm, about 0.06 mm, about 0.07 mm, about 0.1 mm, about 0.12 mm, about 0.15 mm, about 0.17 mm, about 0.2 mm, about 0.22 mm, about 0.25 mm, about 0.27 mm, about 0.3 mm, about 0.32 mm, about 0.35 mm, about 0.37 mm, about 0.4 mm, about 0.42 mm, about 0.45 mm, and about 0.47 mm.

In still further aspects, a suture **100** can comprise filaments made of various materials. In certain aspects, a suture **100** can comprise a polyester material, polyethylene, ultrahigh molecular weight polyethylene, silicone, polyurethane, Teflon, polyether ether ketone (PEEK), or natural tissue, or a combination thereof. In yet further aspects, a suture **100** can comprise engineered materials. In such aspects, the engineered materials can comprise, for example, bicomponent filaments. In some exemplary aspects, bicomponent filaments can be present in core-sheath configuration, side-by-side configuration, or islands-on-sea configurations. It is understood that each component of the bicomponent filaments can be selected depending on the desired application. It is further understood that in certain aspects, a suture **100** comprising a multifilament thread can comprise filaments comprising the same material. While in some alternative aspects, a suture **100** can comprise filaments comprising different materials.

In still further aspects, the one or more components of the prosthetic valve can comprise, for example, and without limitation, leaflets, skirts, support layers, a frame, or any combination thereof.

Some exemplary aspects for the suture's use in the prosthetic valve are shown in the following figures and description.

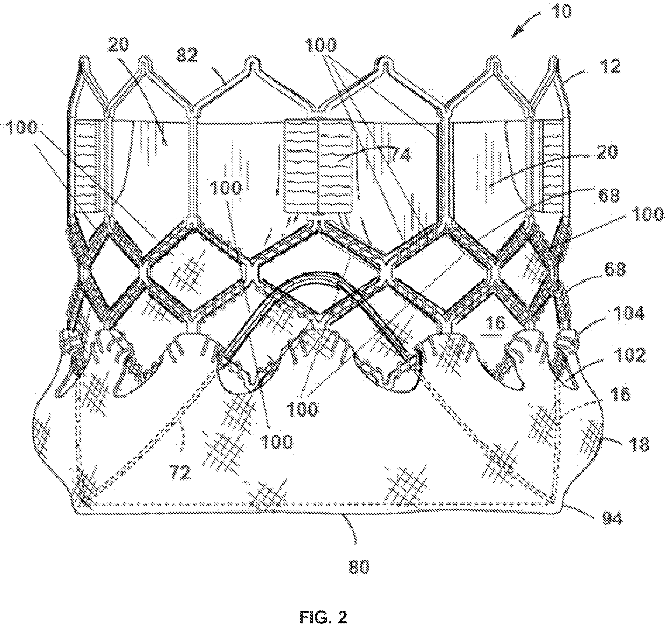

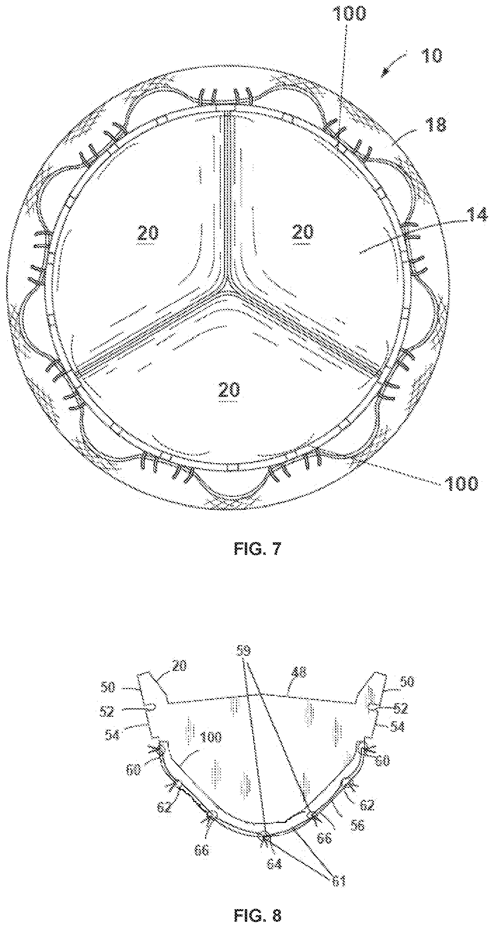

For example, **FIGS. 2** and **7** show side and top plan views, respectively, of an exemplary prosthetic heart valve **10** having an inflow end **80** and an outflow end **82,** according to one aspect. Example prosthetic heart valves **10** are described in U.S. Patent No. 10,463,484, titled "Prosthetic Heart Valve Having Leaflet Inflow Below Frame". The illustrated prosthetic valve **10** is adapted to be implanted in the native aortic annulus, although in other aspects, it can be adapted to be implanted in the other native annuluses of the heart (the mitral valve, pulmonary valve, and tricuspid valve). The prosthetic valve **10** can have one or more of the following components: a stent or frame, **12,** a valvular structure **14,** and an inner skirt, or sealing member, **16.** The valve **10** can also include an outer skirt, or sealing member, **18.** As can be seen in these figures, and as it is discussed in more detail below, suture **100** can be used to attach various portions of the prosthetic valve together. The position of the suture **100** on these figures is only exemplary and non-limiting. Suture **100** can be utilized in any place or position that is configured to couple two or more components of the valve together.

In certain aspects, a prosthetic valve **10** can comprise one or more leaflets, wherein each of the one or more leaflets **20** has a first surface and an opposite second surface, a leaflet inflow edge, and a leaflet outflow edge. In some exemplary aspects and as shown in **FIG. 7****,** the valvular structure **14** can comprise three leaflets **20,** collectively forming a valvular structure, which can be arranged to collapse in a tricuspid arrangement. The lower edge of valvular structure **14** desirably has an undulating, curved scalloped shape. By forming the leaflets **20** with this scalloped geometry, stresses on the leaflets are reduced, which in turn improves the durability of the valve **10.** Moreover, by virtue of the scalloped shape, folds and ripples at the belly of each leaflet **20** (the central region of each leaflet), which can cause early calcification in those areas, can be eliminated or at least minimized. The scalloped geometry also reduces the amount of tissue material used to form the valvular structure **14,** thereby allowing a smaller, more even crimped profile at the inflow end of the valve. The leaflets **20** can be formed of pericardial tissue (e.g., bovine pericardial tissue), biocompatible synthetic materials, or various other suitable natural or synthetic materials as known in the art and described in U.S. Pat. No. 6,730,118.

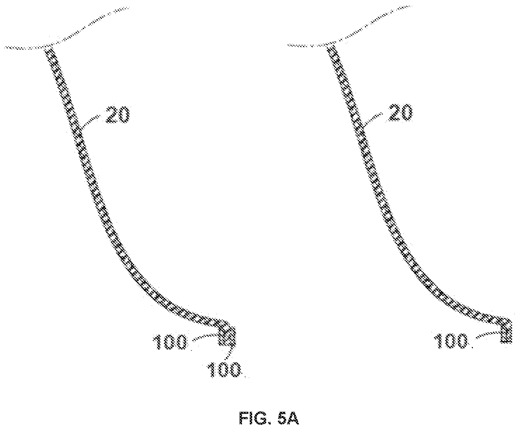

As noted above, valvular structure **14** in the illustrated and unlimited aspect can include three flexible leaflets **20** (although a greater or fewer number of leaflets can be used). An exemplary leaflet **20** is shown in **FIG. 8****.** Each leaflet **20** can have the disclosed herein suture **100** secured with the first attachment member **61** through the plurality of apertures **59** to the first surface of the lower edge portion **56.** Each leaflet **20** in the illustrated configuration can have an upper (outflow) free edge **48** extending between opposing upper tabs **50** on opposite sides of the leaflet **20.** Below each upper tab **50,** there can be a notch **52** separating the upper tab **50** from a corresponding lower tab **54.** The lower (inflow) edge portion **56** of the leaflet extending between respective ends of the lower tabs **54** can include vertical, or axial, edge portions **60** on opposites of the leaflets extending downwardly from corresponding lower tabs **54** and a substantially V-shaped, an intermediate edge portion **62** having a smooth, curved apex portion **64** at the lower end of the leaflet and a pair of oblique portions **66** that extend between the axial edge portions **60** and the apex portion **64.** The oblique portions **66** can have a greater radius of curvature than the apex portion **64.** The leaflets can have various other shapes and/or configurations in other aspects. For example, the leaflets need not have a V-shaped or scalloped inflow edge, and instead, each leaflet can have a square or rectangular shape defining a straight inflow edge. Presence of suture **100** can protect the pericardial tissue of the leaflets **20** from undesirable damages. Certain exemplary aspects of the suture **100** positioning are shown in **FIG. 5A****.** In **FIG. 5A** right side, the suture **100** is positioned on the first surface of the lower edge portion of the leaflet, while in **FIG. 5A** left side, the suture **100** is positioned both on the first and the opposite second surface of the lower edge portion of the leaflet such that the leaflet is sandwiched between two sutures. The sutures in this aspect can be the same or different and can comprise any shape, size, or design of the plurality of apertures as disclosed herein.

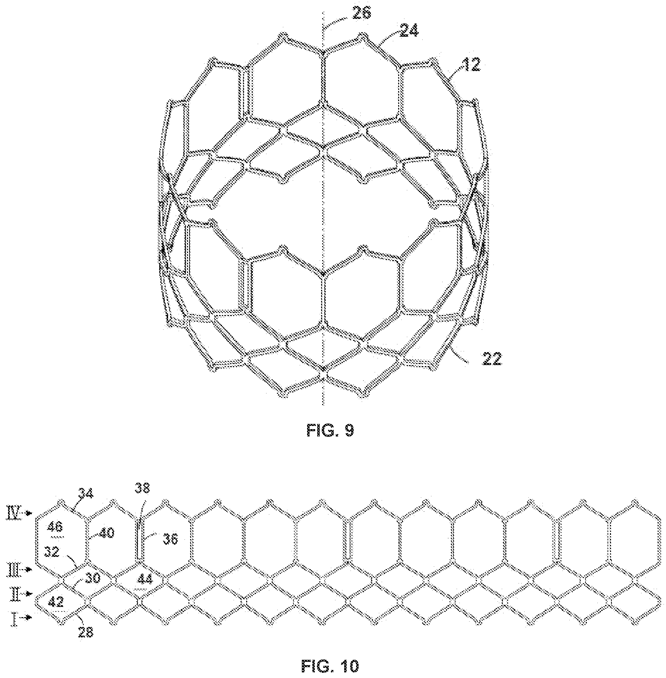

The exemplary leaflets **20** are coupled with the frame of the prosthetic valve **12** (as shown, for example, in F**IGS. 2-4**). The exemplary bare frame **12** is shown in **FIGS. 9** and **10****.** The frame **12** has an inflow end **22,** an outflow end **24,** and a central longitudinal axis **26** extending from the inflow end **22** to the outflow end **24.** The frame **12** can be made of any of various suitable plastically-expandable materials (e.g., stainless steel, etc.) or self-expanding materials (e.g., Nitinol) as known in the art. When constructed of a plastically-expandable material, the frame **12** (and thus the prosthetic valve **10**) can be crimped to a radially compressed state on a delivery catheter and then expanded inside a patient by an inflatable balloon or equivalent expansion mechanism. When constructed of a self-expandable material, the frame **12** (and thus the prosthetic valve **10**) can be crimped to a radially compressed state and restrained in the compressed state by insertion into a sheath or equivalent mechanism of a delivery catheter. Once inside the body, the valve can be advanced from the delivery sheath, which allows the valve to expand to its functional size. Suitable plastically-expandable materials that can be used to form the frame **12** include, without limitation, stainless steel, a nickel-based alloy (e.g., a cobalt-chromium or a nickel-cobalt-chromium alloy), polymers, or combinations thereof. In particular aspects, frame **12** can be made of a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight. It has been found that the use of MP35N to form frame **12** provides superior structural results over stainless steel. In particular, when MP35N is used as the frame material, less material is needed to achieve the same or better performance in radial and crush force resistance, fatigue resistance, and corrosion resistance. Moreover, since less material is required, the crimped profile of the frame can be reduced, thereby providing a lower profile valve assembly for percutaneous delivery to the treatment location in the body.

As further shown in **FIGS. 9** and **10****,** frame **12** in some exemplary and unlimiting aspects can comprise a first, lower row I of angled struts **28** arranged end-to-end and extending circumferentially at the inflow end of the frame; a second row II of circumferentially extending angled struts **30;** a third row III of circumferentially extending, angled struts **32;** and a fourth row IV of circumferentially extending angled struts **34** at the outflow end of the frame **12.** The fourth row IV of angled struts **34** can be connected to the third row III of angled struts **32** by a plurality of axially extending window frame portions **36** (which define commissure windows **38**) and a plurality of axially extending struts **40.** Each axial strut **40** and each frame portion **36** extends from a location defined by the convergence of the lower ends of two angled struts **34** to another location defined by the convergence of the upper ends of two angled struts **32.**

Each commissure window frame portion **36** mounts a respective commissure **74** of the leaflet structure **14.** As can be seen, each frame portion **36** is secured at its upper and lower ends to the adjacent rows of struts to provide a robust configuration that enhances fatigue resistance under cyclic loading of the valve compared to known cantilevered struts for supporting the commissures of the leaflet structure. This configuration enables a reduction in the frame wall thickness to achieve a smaller crimped diameter of the valve. In particular aspects, the thickness of the frame **12** measured between the inner diameter and outer diameter is about 0.48 mm or less.

The struts and frame portions of the frame collectively define a plurality of open cells of the frame. At the inflow end **22** of the frame **12,** struts **28** and struts **30** define a lower row of cells defining openings **42.** The second and third rows of struts **30** and **32,** respectively, define an intermediate row of cells defining openings **44.** The third and fourth rows of struts **32** and **34,** along with frame portions **36** and struts **40,** define an upper row of cells defining openings 46. The openings **46** are relatively large and are sized to allow portions of the valvular structure **14** to protrude, or bulge, into and/or through the openings **46** when the frame **12** is crimped in order to minimize the crimping profile.

The frame **12** can have other configurations or shapes in other aspects. For example, the frame **12** can comprise a plurality of circumferential rows of angled struts **28, 30, 32, 34** connected directly to each other without vertical struts **40** or frame portions **36** between adjacent rows of struts, or the rows of struts **28, 30, 32, 34** can be evenly spaced with vertical struts **40** and/or frame portions **36** extending therebetween. In other aspects, the frame can comprise a braided structure braided from one or more metal wires.

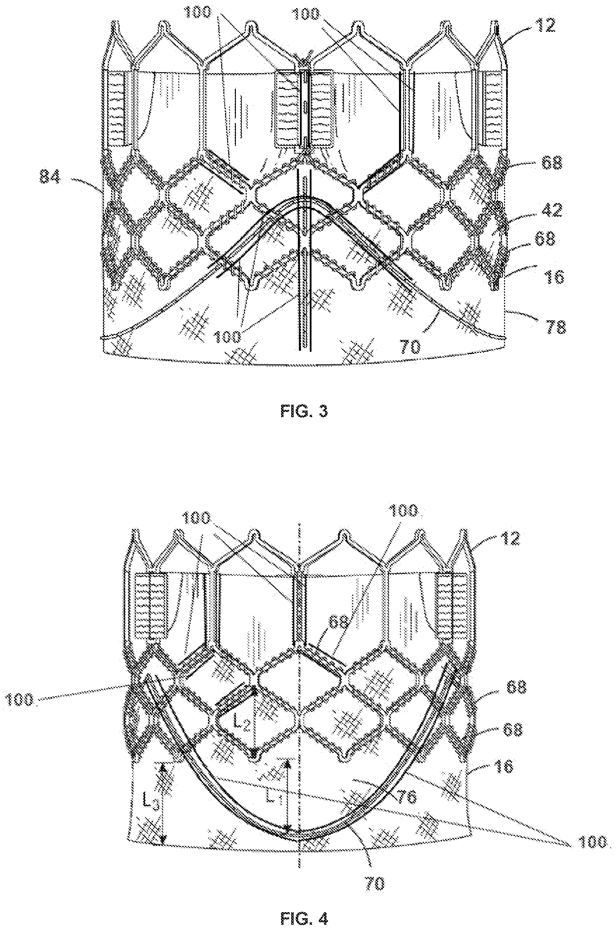

The leaflets **20** can be sutured together to form the assembled valvular structure **14,** which can then be secured to the frame **12.** It is understood that when the leaflets **20** are sutured together, each of the leaflets can comprise a disclosed herein suture **100** having a plurality of apertures **104.** The coupling of the two or more sutures together can be done by passing the first attachment member through the plurality of apertures. It is understood that the suture can be positioned on the first and/or second surface of the leaflet. In still further aspects, when two or more leaflets are coupled together, for example, the sutures can be positioned in parallel to each other. Generally, it is understood that the suture can be positioned in any location that requires passing the attachment member through the leaflet or any other tissue. In certain aspects and as described herein, the disclosed sutures are designed to reinforce the tissue and prevent its tear. For example, the leaflets **20** can be secured to one another at their adjacent sides to form commissures **74** of the valvular structure. As can be seen in **FIG. 3****,** a suture **100** can be used to form such commissures. A plurality of flexible connectors (not shown) can be used to interconnect pairs of adjacent sides of the leaflets and to mount the leaflets to the commissure window frame portions **30,** as further discussed below. It is further understood that such a mounting can be done by the use of the sutures when the flexible connectors are passed through the plurality of apertures present in the leaflet. The leaflets **20** can additionally and/or alternatively be secured together via adjacent sub-commissure portions (not shown) of two leaflets that can be sutured directly to each other with the use of one or more sutures described herein.

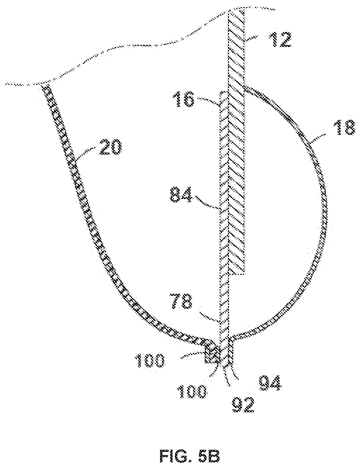

In still further exemplary aspects, the one or more leaflets **20** can be attached to the inner skirt **16,** as shown, for example, in **FIG. 5B****.** In such aspects, to reinforce the leaflets and to prevent any undesirable damages, the disclosed herein suture **100** can be positioned along the lower (inflow) edge portions **56** of the leaflets **20.** The reinforcement suture **100** enables a secure suturing and protects the pericardial tissue of the valvular structure **14** from tears. Valvular structure **14** can be sandwiched between the inner skirt **16** and the suture **100.** The first attachment member **70 (****FIG. 3****)** secures the suture **100** (**FIG. 3**) to inner skirt **16.** It is understood that the first attachment member can be any member described herein and can comprise stitches such as, for example, an Ethibond suture. The attachment member **70** can track the curvature of the bottom edge of valvular structure **14** and are collectible referred to as scallop line **72.** In other exemplary and unlimiting aspects, in lieu of or in addition to the sutures **100** along the inner surface of the leaflets, sutures **100** can be positioned between the inner skirt **16** and the inflow edge portions **(****FIG. 5B****)**.

The inner skirt **16** can have a plurality of functions, which can include assisting in securing the valvular structure **14** and/or the outer skirt to the frame **12** and to assist in forming a good seal between the valve **10** and the native annulus by blocking the flow of blood below the lower edges of the leaflets. The inner skirt **16** can comprise a tough, tear-resistant material such as polyethylene terephthalate (PET), although various other synthetic or natural materials can be used. The thickness of the skirt desirably is less than 6 mils or 0.15 mm, and desirably less than 4 mils or 0.10 mm, and even more desirably about 2 mils or 0.05 mm. In particular aspects, the skirt **16** can have a variable thickness, for example, the skirt can be thicker at its edges than at its center. In one implementation, the skirt **16** can comprise a PET skirt having a thickness of about 0.07 mm at its edges and about 0.06 mm at its center. The thinner skirt can provide for better crimping performances while still providing good perivalvular sealing.

**FIGS. 3** and **4** show the frame **12,** the valvular structure **14** and the inner skirt **16** after securing the valvular structure **14** to the inner skirt **16** with the use of suture **100** and then securing these components to the frame **12** also with the use of the sutures **100.** Inner skirt **16** can be secured to the inside of frame **12** via sutures **68.** Suture **100** can be utilized to mitigate damage to the inner skirt **16.** As shown in **FIGS. 3-4****,** portions of the inflow edge **56** of the leaflets **20** are positioned between diagonal lines defined by the struts **28, 30** of the first and second circumferential rows of the struts (as shown in **FIGS. 9** and **10**). In some aspects, the inflow edges **56** of the leaflets **20** can track the diagonal lines defined by the struts **28, 30.** In such aspects, the portions of the inflow edge **56** above the inflow end of the frame can be coupled to adjacent struts **28, 30** by use of the disclosed sutures **100.**

The outflow end portion of the valvular structure **14** can be secured to the window frame portions **36** with sutures **100.** In particular, each leaflet **20** can have opposing tab portions, each of which is paired with an adjacent tab portion of another leaflet to form a commissure **74.** It is understood that in such aspect, each of the tab portions of the leaflet is coupled with the adjacent tab portion of another leaflet with the use of suture **100.** It is further understood that in such aspects, one or more sutures **100** can be present. In some aspects, each tab can be sandwiched between at least two sutures **100,** or the suture can be positioned only on one surface of each tab. It is further understood that two adjacent tabs can have sutures positioned on the same or different surfaces of the tab. As shown in **FIG. 2****,** the commissures **74** can extend through windows **38** of respective window frame portions **36** and be retained in place with the attachment member passing through the leaflet and the suture **100.** Further details of the commissures **74** and a method for assembling the commissures and mounting them to the frame are disclosed in U.S. Publication No. 2012/0123529.

Inner skirt **16** can terminate short of the window frame portions **36** and does not extend the entire length of the frame **12.** In alternative aspects, inner skirt **16** can extend the entire length or substantially the entire length of the frame **12** from below the inflow end **22** to the outflow end **24.** In other aspects, inner skirt **16** can extend up to the second row of angled struts **30.**

In particular aspects, and as shown in **FIGS. 2-4****,** the valvular structure **14** can extend axially beyond the inflow end **22** of the frame **12** such that at least a portion of the inflow edges **56** of each leaflet is not supported by the frame **12.** For example, the apex portions **64** of the leaflets **20** can extend axially beyond and can be unsupported by the frame **12.** Additionally and/or alternatively, the oblique portions **66** and the apex portions **64** can extend axially beyond and can be unsupported by the frame **12.** Additionally and/or alternatively, the intermediate portions **62,** the oblique portions **66** and the apex portions **64** can extend axially beyond and can be unsupported by the frame **12.** Additionally and/or alternatively, the vertical edge portions **60,** the intermediate portions **62,** the oblique portions **66** and the apex portions **64** can extend axially beyond and can be unsupported by the frame **12.** In some aspects, a portion **76** of each leaflet extends below the inflow end **22** of the frame a length L₁ that is equal to or greater than a length L₂ of each cell opening **42** in the axial direction. In some aspects, L₁ can range between about 2 mm to 8 mm in length, with 4 mm being a specific example. Additionally and/or alternatively, L₁ can be up to 12 mm. In some aspects, L₂ can range between about 3 mm to 5 mm in length, with 4 mm being a specific example. Additionally and/or alternatively, L₂ can range between 2 mm to 7 mm.

Additionally and/or alternatively, as shown in **FIG. 5B****,** the inner skirt **16** can include a first portion **84,** also referred to as supported portion, that extends circumferentially around the central longitudinal axis of the prosthetic valve along an inner surface of the frame **12** and a second portion, also referred to as unsupported portion **78,** at the inflow end **80** of the prosthetic valve **10.** The unsupported portion **78** is unsupported by the frame **12.** The unsupported portion **78** can extend circumferentially around the central longitudinal axis of the prosthetic valve to form an unsupported circumference at the inflow end **80.** As shown in **FIG. 5B****,** the portion of each leaflet **20** that is not supported by the frame **12** can be connected to the inner skirt **16.** In some aspects, a length L₃ of the unsupported portion **78** is equal to or greater than the length L₂ of the openings **42.** In some aspects, L₃ can be 2 mm to 3 mm longer than L₁. Additionally and/or alternatively, L₃ can range from the same length as L₁ to 5 mm longer than L₁. In some aspects, L₃ can range between about 2 mm to 11 mm in length, with 6 mm being a specific example. Additionally and/or alternatively, L₃ can be up to 15 mm.

In certain aspects and as described herein, the prosthetic valve can also comprise an outer skirt **18.** The outer skirt **18** can be laser cut or otherwise formed from a strong, durable piece of material, such as woven PET, although other synthetic or natural materials can be used. The outer skirt **18** can be secured to the lower edge of the inner skirt **16** at the inflow end of the prosthetic valve, such as by sutures, welding, and/or an adhesive with or without sutures **100.** In particular aspects, the lower edge of the outer skirt **18** is tightly sutured (with or without sutures **100**) or otherwise secured (e.g., by welding or an adhesive) to the inner skirt **16.** Again, it is further understood that in each coupling location, the disclosed suture **100** can be utilized.

The absence of metal components of the frame or other rigid members along the inflow end portion of the prosthetic valve advantageously reduces mechanical compression of the native valve annulus (e.g., the aortic annulus) and the left ventricular outflow tract (when implanted in the aortic position), thus reducing the risk of trauma to the surrounding tissue.

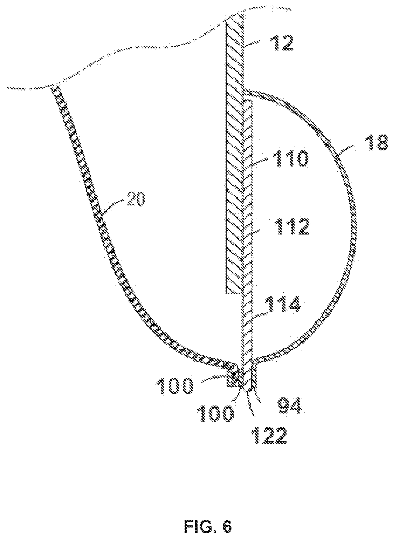

As shown in **FIG. 6****,** some aspects of a prosthetic valve **10** do not include an inner skirt **16** and instead can include a support layer **110** disposed radially between the frame **12** and the outer skirt **18.** In such aspects, the support layer **110** and the outer skirt **18** can form an outer sealing member with the support layer **110,** forming an inner wall or layer of the sealing member and the outer skirt forming an outer wall or layer of the sealing member A first portion **112** of the support layer **110** can extend circumferentially around the central longitudinal axis of the prosthetic valve along an outer surface of the frame **12,** and a second portion **114** of the support layer **110** can extend circumferentially around the central longitudinal axis axially beyond the inflow end **22** of the frame. The unsupported portions **76** of the leaflets can be connected to the support layer **110** (e.g., by sutures, an adhesive, and/or welding) similar to the manner that the leaflets are connected to the inner skirt in the aspect **of** FIG. 5A-B. More specifically, in such aspects, the inflow edge portions **56** is sutured to sutures **100** and the support layer **110** with the inflow edge portions **56** sandwiched between the support layer **110** and the suture **100.** Additionally, and/or alternatively, sutures **100** can be positioned between the inflow edge portions **56** and the support layer **110,** with sutures securing these layers together. Additionally and/or alternatively, the outer skirt **18** can be connected to the second portion **114** of the support layer with or without suture **100** (e.g., by sutures, an adhesive, and/or welding).

It is further understood that the attachment members described herein can comprise a first attachment member, a second attachment member, or any other attachment member. It is further understood that the numbering describing the attachment member is used only to demonstrate that the attachment member can be the same or different. For example, in some aspects, the coupling of the suture **100** with any component of the valve can be done with the stitches comprising the same material or the stitches comprising different materials. In yet other aspects, the coupling can be done with the stitches, flexible connectors, wires, or any other known in the art attachment members appropriate for the desired application.

The aspects of the present disclosure also relate to methods of making a prosthetic valve comprising providing at least one reinforcement member; attaching the at least one reinforcement member with a first attachment member to one or more components of a prosthetic valve; wherein the at least one reinforcement member comprises a suture having a longitudinal axis and a transverse axis perpendicular to the longitudinal axis, and wherein at least a portion of the suture comprises a plurality of apertures, wherein each of the plurality of apertures is configured to receive the first attachment member for coupling the suture to the prosthetic valve.

It is understood that any sutures described herein can be used to produce prosthetic valves. In certain aspects, the plurality of apertures are braided within the suture. In such aspects, the plurality of apertures are formed by braiding. In yet other aspects, the plurality of apertures are formed by combining two or more threads and fusing them together such that the desired apertures are formed. It is further understood that in the methods disclosed herein, the prosthetic valve can comprise any of the components described above. It is further understood that any of the described above attachment members can be used to couple the sutures to any of the prosthetic valve components.

Also disclosed herein is a kit comprising: at least one suture having a longitudinal axis and a transverse axis perpendicular to the longitudinal axis, and wherein at least a portion of the suture comprises a plurality of apertures, wherein each of the plurality of apertures is configured to receive an attachment member for coupling the suture to an article. It is understood that these kits can be used in any known application where the coupling of two or more members is required. In still further aspects, the disclosed herein kit can be used in any known application where the need to protect coupling members from the tears or any other damage exists. For example and without limitation, the articles the suture is configured to be coupled with can comprise a fabric, a tissue, a prosthetic valve, or any combination thereof. In still further aspects, the suture present in the kit is configured to be a reinforcement member. While in other aspects, the suture does not behave as a reinforcement member but is just used as a member configured to couple various components of the article.

Any described herein sutures can be present in the disclosed kits. In certain aspects, the first attachment can also be present in the kit. While in other aspects, the first attachment member is not a part of the kit, and any known in the art attachment members that are suitable for the desired application can be used. In certain aspects, the first attachment member can comprise one or more stitches, flexible connectors, or any combination thereof. In yet further aspects, the suture present in the kit comprises a centerline positioned along its longitudinal axis. In some exemplary aspects, the plurality of apertures can be positioned within at least a portion of the centerline of the suture. While in other aspects, the plurality of apertures can be positioned along all length of the suture. In alternative aspects, each of the plurality of apertures is spaced apart by a spacing increment d along a length of the suture. It is understood that in some aspects, the spacing increment d between any two adjacent apertures of the plurality of apertures is the same. While in other aspects, the spacing increment d between any two adjacent apertures of the plurality of apertures is different. In yet further aspects, the suture present in the kit can comprise a polyester material, polyethylene, ultrahigh molecular weight polyethylene, silicone, polyurethane, Teflon, polyether ether ketone (PEEK), or natural tissue, or a combination thereof. In still further aspects, the suture comprises a plurality of apertures that can be braided within the suture. While in other aspects, the suture can comprise two or more cords fused together to form the plurality of apertures.

In view of the many possible aspects to which the principles of the disclosed disclosure can be applied, it should be recognized that the illustrated aspects are only some examples of the disclosure and should not be taken as limiting the scope of the disclosure. Rather, the scope of the disclosure is defined by the following claims.

## Claims

1. A prosthetic heart valve comprising:
at least one reinforcement member (100) configured to be attached with a first attachment member (61, 70) to one or more components of the prosthetic heart valve;
wherein the at least one reinforcement member (100) comprises a suture having a longitudinal axis (103) and a transverse axis (101) perpendicular to the longitudinal axis (103);
wherein at least a portion of the suture comprises a plurality of apertures (104);
wherein each of the plurality of apertures (104) is configured to receive the first attachment member (61, 70) for coupling the suture to the prosthetic heart valve; and
wherein the plurality of apertures (104) is braided within the suture or wherein the suture comprises two or more cords fused together to form the plurality of apertures (104).

2. The prosthetic heart valve of claim 1, wherein the first attachment member (61, 70) comprises one or more stitches.

3. The prosthetic heart valve of claim 1 or 2, wherein the suture comprises:
a) a centerline (102) positioned along its longitudinal axis (103),
optionally wherein the plurality of apertures (104) are positioned within at least a portion of the centerline (102) of the suture; and/or
b) a polyester material, polyethylene, ultrahigh molecular weight polyethylene, silicone, polyurethane, Teflon, polyether ether ketone (PEEK), natural tissue, or a combination thereof.

4. The prosthetic heart valve of any one of claims 1-3, wherein each of the plurality of apertures (104) is spaced apart by a spacing increment d along a length of the suture,
optionally wherein the spacing increment d between any two adjacent apertures (104) of the plurality of apertures (104) is:
a) the same; or
b) different.

5. The prosthetic heart valve of any preceding claim, wherein the one or more components of the prosthetic heart valve comprise leaflets (20), skirts, support layers, a frame (12), or any combination thereof.

6. The prosthetic heart valve claim 5, wherein the frame (12) comprises an annular frame (12) having an inflow end (22), an outflow end (24) and a central longitudinal axis (26) extending from the inflow end (22) to the outflow end (24).

7. The prosthetic heart valve of claim 5 or claim 6, wherein each of leaflets (20) has a first surface and an opposite second surface, a leaflet inflow edge (56), and a leaflet outflow edge,
optionally wherein the leaflet inflow edge (56) is positioned at least partially outside of the frame (12).

8. The prosthetic heart valve of claim 7, wherein the leaflet outflow edge is positioned within the frame (12).

9. The prosthetic heart valve of claim 7 or claim 8, wherein at least a portion of the leaflet inflow edges (56) is unsupported by the frame (12).

10. The prosthetic heart valve of any one of claims 7 to 9, wherein the at least one reinforcement member (100) is positioned adjacent to the first surface or the second surface of the one or more leaflets (20) along the leaflet inflow edge (56).

11. The prosthetic heart valve of any of claims 7 to 10, wherein the at least one reinforcement member (100) comprises two reinforcement members (100), including a first reinforcement member (100) positioned adjacent to the first surface of the one or more leaflets (20) along the leaflet inflow edge (56) and a second reinforcement member (100) positioned adjacent to the second surface of the one or more leaflets (20) along the leaflet inflow edge (56), such that the one or more leaflets (20) are sandwiched between two reinforcement members (100).

12. The prosthetic heart valve of any of claims 7 to 11, wherein the valve comprises at least two leaflets (20), and wherein each two of adjacent leaflets (20) are secured to each other,
optionally wherein the at least one reinforcement member (100) is positioned adjacent to the first or second surface of each of the at least two leaflets (20) such that the adjacent leaflets (20) are secured to each other with the attachment member (100) passing through the plurality of apertures (104) of the suture.

13. The prosthetic heart valve of any one of claims 6 to 12, further comprising an inner skirt (16) having a first portion (84) that extends circumferentially around the central longitudinal axis (26) along an inner surface of the frame (12) and is supported by the frame (12) and a second portion (78) that extends circumferentially around the central longitudinal axis (26) outside of the frame (12) and is not supported by the frame (12),
optionally wherein the reinforcement member (100) is positioned between the one or more leaflets (20) and at least a portion of the inner skirt (16) such that the one or more leaflets (20) are secured with the at least a portion of the inner skirt (16) with the attachment member (00) passing through the plurality of apertures (104); and
optionally wherein the one or more leaflets (20) are sandwiched between the at least two reinforcement members (100) and at least a portion of the inner skirt (16).

14. The prosthetic heart valve of claim 7 or claim 8, wherein each of the one or more leaflets (20) is secured to the one or more components of the prosthetic heart valve with at least one reinforcement member (100).

15. A method of making a prosthetic heart valve comprising:
providing at least one reinforcement member (100);
attaching the at least one reinforcement member (100) with a first attachment member (61, 70) to one or more components of a prosthetic heart valve;
wherein the at least one reinforcement member (100) comprises a suture having a longitudinal axis (103) and a transverse axis perpendicular to the longitudinal axis (103);
wherein at least a portion of the suture comprises a plurality of apertures (104);
wherein each of the plurality of apertures (104) is configured to receive the first attachment member (61, 70) for coupling the suture to the prosthetic heart valve; and
wherein the plurality of apertures (104) is braided within the suture or wherein the suture comprises two or more cords fused together to form the plurality of apertures (104).

## Patentansprüche

1. Herzklappenprothese, umfassend:
mindestens ein Verstärkungsglied (100), das dazu ausgestaltet ist, mit einem ersten Anbringglied (61, 70) an einer oder mehreren Komponenten der Herzklappenprothese angebracht zu werden,
wobei das mindestens eine Verstärkungsglied (100) eine Naht mit einer Längsachse (103) und einer senkrecht zu der Längsachse (103) verlaufenden Querachse (101) umfasst,
wobei mindestens ein Abschnitt der Naht eine Vielzahl von Öffnungen (104) umfasst,
wobei jede der Vielzahl von Öffnungen (104) zur Aufnahme des ersten Anbringglieds (61, 70) ausgestaltet ist, um die Naht an die Herzklappenprothese zu koppeln, und
wobei die Vielzahl von Öffnungen (104) in der Naht geflochten sind oder wobei die Naht zwei oder mehr Kordeln umfasst, die zum Bilden der Vielzahl von Öffnungen (104) verschmolzen sind.

2. Herzklappenprothese nach Anspruch 1, wobei das erste Anbringglied (61, 70) einen oder mehrere Fäden umfasst.

3. Herzklappenprothese nach Anspruch 1 oder 2, wobei die Naht Folgendes umfasst:
a) eine entlang ihrer Längsachse (103) positionierte Mittellinie (102),
optional wobei die Vielzahl von Öffnungen (104) in mindestens einem Abschnitt der Mittellinie (102) der Naht positioniert sind und/oder
b) ein Polyestermaterial, Polyethylen, ultrahochmolekulares Polyethylen, Silikon, Polyurethan, Teflon, Polyetheretherketon (PEEK), natürliches Gewebe oder eine Kombination davon.

4. Herzklappenprothese nach einem der Ansprüche 1 - 3, wobei jede der Vielzahl von Öffnungen (104) über einen Abstandszuwachs *d* entlang einer Länge der Naht beabstandet ist,
optional wobei der Abstandszuwachs *d* zwischen zwei beliebigen benachbarten Öffnungen (104) der Vielzahl von Öffnungen (104)
a) der gleiche oder
b) ein anderer
ist.

5. Herzklappenprothese nach einem der vorhergehenden Ansprüche, wobei die eine oder die mehreren Komponenten der Herzklappenprothese Segel (20), Schürzen, Stützschichten, einen Rahmen (12) oder eine Kombination davon umfassen.

6. Herzklappenprothese nach Anspruch 5, wobei der Rahmen (12) einen ringförmigen Rahmen (12) umfasst, der ein Einströmende (22), ein Ausströmende (24) und eine mittlere Längsachse (26) aufweist, die sich von dem Einströmende (22) zu dem Ausströmende (24) erstreckt.

7. Herzklappenprothese nach Anspruch 5 oder Anspruch 6, wobei jedes der Segel (20) eine erste Fläche und eine gegenüberliegende zweite Fläche, eine Segelanströmkante (56) und eine Segelabströmkante hat,
optional wobei die Segelanströmkante (56) mindestens teilweise außerhalb des Rahmens (12) positioniert ist.

8. Herzklappenprothese nach Anspruch 7, wobei die Segelabströmkante in dem Rahmen (12) positioniert ist.

9. Herzklappenprothese nach Anspruch 7 oder Anspruch 8, wobei mindestens ein Teil der Segelanströmkanten (56) nicht von dem Rahmen (12) gestützt ist.

10. Herzklappenprothese nach einem der Ansprüche 7 bis 9, wobei das mindestens eine Verstärkungsglied (100) der ersten Fläche oder der zweiten Fläche des einen oder der mehreren Segel (20) benachbart entlang der Segelanströmkante (56) positioniert ist.

11. Herzklappenprothese nach einem der Ansprüche 7 bis 10, wobei das mindestens eine Verstärkungsglied (100) zwei Verstärkungsglieder (100) umfasst, einschließlich eines ersten Verstärkungsglieds (100), das der ersten Fläche des einen oder der mehreren Segel (20) benachbart entlang der Segelanströmkante (56) positioniert ist, und eines zweiten Verstärkungsglieds (100), das der zweiten Fläche des einen oder der mehreren Segel (20) benachbart entlang der Segelanströmkante (56) positioniert ist, so dass das eine oder die mehreren Segel (20) zwischen zwei Verstärkungsgliedern (100) liegen.

12. Herzklappenprothese nach einem der Ansprüche 7 bis 11, wobei die Klappe mindestens zwei Segel (20) umfasst und wobei jeweils zwei benachbarte Segel (20) aneinander befestigt sind,
optional wobei das mindestens eine Verstärkungsglied (100) der ersten oder zweiten Fläche jedes der mindestens zwei Segel (20) benachbart positioniert sind, so dass die benachbarten Segel (20) mit dem Anbringglied (100) aneinander befestigt sind, das durch die Vielzahl von Öffnungen (104) in der Naht geht.

13. Herzklappenprothese nach einem der Ansprüche 6 bis 12, ferner umfassend eine innere Schürze (16) mit einem ersten Abschnitt (84), der sich umfangsmäßig um die mittlere Längsachse (26) entlang einer Innenfläche des Rahmens (12) erstreckt und von dem Rahmen (12) gestützt wird, und einem zweiten Abschnitt (78), der sich umfangsmäßig um die mittlere Längsachse (26) außerhalb des Rahmens (12) erstreckt und nicht von dem Rahmen (12) gestützt wird,
optional wobei das Verstärkungsglied (100) zwischen dem einen oder den mehreren Segeln (20) und mindestens einem Abschnitt der inneren Schürze (16) positioniert ist, so das das eine oder die mehreren Segel (20) mit dem mindestens einen Abschnitt der inneren Schürze (16) mit dem Anbringglied (00) befestigt sind, das durch die Vielzahl von Öffnungen (104) geht, und
optional wobei das eine oder die mehreren Segel (20) zwischen den mindestens zwei Verstärkungsgliedern (100) und mindestens einem Abschnitt der inneren Schürze (16) liegen.

14. Herzklappenprothese nach Anspruch 7 oder Anspruch 8, wobei jedes des einen oder der mehreren Segel (20) mit mindestens einem Verstärkungsglied (100) an der einen oder den mehreren Komponenten der Herzklappenprothese befestigt ist.

15. Verfahren zur Herstellung einer Herzklappenprothese, umfassend:
Bereitstellen von mindestens einem Verstärkungsglied (100),
Anbringen des mindestens einen Verstärkungsglieds (100) mit einem ersten Anbringglied (61, 70) an einer oder mehreren Komponenten einer Herzklappenprothese,
wobei das mindestens eine Verstärkungsglied (100) eine Naht mit einer Längsachse (103) und einer senkrecht zu der Längsachse (103) verlaufenden Querachse (101) umfasst,
wobei mindestens ein Abschnitt der Naht eine Vielzahl von Öffnungen (104) umfasst,
wobei jede der Vielzahl von Öffnungen (104) zur Aufnahme des ersten Anbringglieds (61, 70) ausgestaltet ist, um die Naht an die Herzklappenprothese zu koppeln, und
wobei die Vielzahl von Öffnungen (104) in der Naht geflochten sind oder wobei die Naht zwei oder mehr Kordeln umfasst, die zum Bilden der Vielzahl von Öffnungen (104) verschmolzen sind.

## Revendications

1. Valve cardiaque prothétique comprenant :
au moins un élément de renforcement (100) conçu pour être attaché avec un premier élément d'attache (61, 70) à un ou plusieurs composants de la valve cardiaque prothétique ;
dans laquelle l'au moins un élément de renforcement (100) comprend un élément de suture présentant un axe longitudinal (103) et un axe transversal (101) perpendiculaire à l'axe longitudinal (103) ;
dans laquelle au moins une partie de l'élément de suture comprend une pluralité d'ouvertures (104) ;
dans laquelle chaque ouverture de la pluralité d'ouvertures (104) est conçue pour recevoir le premier élément d'attache (61, 70) pour accoupler l'élément de suture à la valve cardiaque prothétique ; et
dans laquelle la pluralité d'ouvertures (104) est formée par tressage au sein de l'élément de suture ou dans laquelle l'élément de suture comprend au moins deux fils assemblés par fusion pour former la pluralité d'ouvertures (104).

2. Valve cardiaque prothétique selon la revendication 1, dans laquelle le premier élément d'attache (61, 70) comprend un ou plusieurs liens.

3. Valve cardiaque prothétique selon la revendication 1 ou 2, dans laquelle l'élément de suture comprend :
a) une ligne médiane (102) située le long de son axe longitudinal (103),
éventuellement dans laquelle la pluralité d'ouvertures (104) sont situées sur au moins une partie de la ligne médiane (102) de l'élément de suture ; et/ou
b) un matériau de polyester, un polyéthylène, un polyéthylène de masse moléculaire très élevée, une silicone, un polyuréthane, du Téflon, une polyétheréthercétone, un tissu naturel ou une combinaison de ceux-ci.

4. Valve cardiaque prothétique selon l'une quelconque des revendications 1 à 3, dans laquelle la pluralité d'ouvertures (104) sont mutuellement espacées par un intervalle d le long d'une longueur de l'élément de suture,
éventuellement dans laquelle l'intervalle d entre deux ouvertures (104) adjacentes quelconques parmi la pluralité d'ouvertures (104) est :
a) égal ; ou
b) différent.

5. Valve cardiaque prothétique selon l'une quelconque des revendications précédentes, dans laquelle le ou les composants de la valve cardiaque prothétique comprennent des feuillets (20), des jupes, des couches de support, une structure (12) ou une combinaison quelconque de ceux-ci .

6. Valve cardiaque prothétique selon la revendication 5, dans laquelle la structure (12) comprend une structure annulaire (12) comportant une extrémité d'entrée (22), une extrémité de sortie (24) et un axe longitudinal central (26) s'étendant entre l'extrémité d'entrée (22) et l'extrémité de sortie (24).

7. Valve cardiaque prothétique selon la revendication 5 ou la revendication 6, dans laquelle chacun des feuillets (20) comporte une première surface et une seconde surface opposée, un bord d'entrée de feuillet (56) et un bord de sortie de feuillet,
éventuellement dans laquelle le bord d'entrée de feuillet (56) est situé au moins partiellement à l'extérieur de la structure (12).

8. Valve cardiaque prothétique selon la revendication 7, dans laquelle le bord de sortie de feuillet est situé à l'intérieur de la structure (12).

9. Valve cardiaque prothétique selon la revendication 7 ou la revendication 8, dans laquelle au moins une partie des bords d'entrée de feuillet (56) n'est pas supportée par la structure (12).

10. Valve cardiaque prothétique selon l'une quelconque des revendications 7 à 9, dans laquelle l'au moins un élément de renforcement (100) est placé près de la première surface ou de la seconde surface du ou des feuillets (20) le long du bord d'entrée de feuillet (56).

11. Valve cardiaque prothétique selon l'une quelconque des revendications 7 à 10, dans laquelle l'au moins un élément de renforcement (100) comprend deux éléments de renforcement (100), comprenant un premier élément de renforcement (100) placé près de la première surface du ou des feuillets (20) le long du bord d'entrée de feuillet (56) et un second élément de renforcement (100) placé près de la seconde surface du ou des feuillets (20) le long du bord d'entrée de feuillet (56), de telle sorte que le ou les feuillets (20) soient pris en sandwich entre deux éléments de renforcement (100).

12. Valve cardiaque prothétique selon l'une quelconque des revendications 7 à 11, la valve comprenant au moins deux feuillets (20), et les feuillets (20) de chaque paire de feuillets adjacents étant fixés l'un à l'autre,
éventuellement dans laquelle l'au moins un élément de renforcement (100) est placé près de la première ou la seconde surface de chacun des au moins deux feuillets (20) de telle sorte que les feuillets (20) adjacents soient fixés l'un à l'autre avec l'élément d'attache (100) passant à travers la pluralité d'ouvertures (104) de l'élément de suture.

13. Valve cardiaque prothétique selon l'une quelconque des revendications 6 à 12, comprenant, en outre, une jupe intérieure (16) comportant une première partie (84) qui s'étend de manière circonférentielle autour de l'axe longitudinal central (26) le long d'une surface intérieure de la structure (12) et est supportée par la structure (12) et une seconde partie (78) qui s'étend de manière circonférentielle autour de l'axe longitudinal central (26) à l'extérieur de la structure (12) et n'est pas supportée par la structure (12),
éventuellement dans laquelle l'élément de renforcement (100) est placé entre le ou les feuillets (20) et au moins une partie de la jupe intérieure (16) de telle sorte que le ou les feuillets (20) soient fixés à l'au moins une partie de la jupe intérieure (16) avec l'élément d'attache (00) passant à travers la pluralité d'ouvertures (104) ; et
éventuellement dans laquelle le ou les feuillets (20) sont pris en sandwich entre les au moins deux éléments de renforcement (100) et au moins une partie de la jupe intérieure (16).

14. Valve cardiaque prothétique selon la revendication 7 ou la revendication 8, dans laquelle le ou chacun des feuillets (20) est fixé au(x) composant(s) de la valve cardiaque prothétique avec au moins un élément de renforcement (100).

15. Procédé de fabrication d'une valve cardiaque prothétique comprenant :
préparer au moins un élément de renforcement (100) ;
attacher l'au moins un élément de renforcement (100) avec un premier élément d'attache (61, 70) à un ou plusieurs composants d'une valve cardiaque prothétique ;
dans lequel l'au moins un élément de renforcement (100) comprend un élément de suture présentant un axe longitudinal (103) et un axe transversal perpendiculaire à l'axe longitudinal (103) ;
dans lequel au moins une partie de l'élément de suture comprend une pluralité d'ouvertures (104) ;
dans lequel chaque ouverture de la pluralité d'ouvertures (104) est conçue pour recevoir le premier élément d'attache (61, 70) pour accoupler l'élément de suture à la valve cardiaque prothétique ; et
dans lequel la pluralité d'ouvertures (104) est formée par tressage au sein de l'élément de suture ou dans lequel l'élément de suture comprend au moins deux fils assemblés par fusion pour former la pluralité d'ouvertures (104).
